(19)

**Europäisches Patentamt**
**European Patent Office**
**Office européen des brevets**

(11) **EP 4 700 360 A1**

(12) **EUROPEAN PATENT APPLICATION**
published in accordance with Art. 153(4) EPC

(43) Date of publication:
**25.02.2026 Bulletin 2026/09**

(21) Application number: **24839276.3**

(22) Date of filing: **25.04.2024**

(51) International Patent Classification (IPC):
**G01N 3/00** $^{(2006.01)}$       **G01N 17/00** $^{(2006.01)}$

(52) Cooperative Patent Classification (CPC):
**G01N 3/00; G01N 17/00**

(86) International application number:
**PCT/JP2024/016287**

(87) International publication number:
**WO 2025/013378 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.07.2023 JP 2023114923**

(71) Applicant: **JFE Steel Corporation**
**Tokyo, 100-0011 (JP)**

(72) Inventors:
• **MIYAJIMA Ayaka**
**Tokyo 100-0011 (JP)**

• **OKANO Hiroshi**
**Tokyo 100-0011 (JP)**
• **OTSUKA Shinji**
**Tokyo 100-0011 (JP)**
• **KAWABE Nao**
**Tokyo 100-0011 (JP)**
• **KIM Jingeum**
**Tokyo 100-0011 (JP)**
• **TAKASHIMA Katsutoshi**
**Tokyo 100-0011 (JP)**
• **MATSUDA Hiroshi**
**Tokyo 100-0011 (JP)**

(74) Representative: **Grünecker Patent- und**
**Rechtsanwälte**
**PartG mbB**
**Leopoldstraße 4**
**80802 München (DE)**

(54) **METHOD FOR EVALUATING DELAYED FRACTURE CHARACTERISTICS OF WELD ZONE OF METAL MATERIAL**

(57)    The present invention is directed to providing a method for evaluating delayed fracture properties of a weld of a metal material, which can quantitatively evaluate the delayed fracture properties of the weld with high accuracy.

The method for evaluating delayed fracture properties of a weld of a metal material includes: a hydrogen introduction step of introducing hydrogen into a test material composed of two or more metal materials having a weld; a tensile load application step of applying a tensile load to the test material into which hydrogen has been introduced and acquiring a displacement-load curve; and a first evaluation step of evaluating a crack initiation load based on the displacement-load curve.

FIG. 1

EP 4 700 360 A1

# Description

Technical Field

The present invention relates to a method for evaluating delayed fracture properties of a weld of a metal material.

Background Art

In recent years, from the viewpoint of preventing global warming, there is a demand for improving energy efficiency, for example gasoline fuel efficiency in the case of automobiles, by reducing the weight of moving bodies such as automobiles, ships, and railway cars. A steel material, which is one of constituent materials for moving bodies, is required to have an increased strength in order to ensure the same level of safety even when its thickness is reduced in order to reduce its weight. On the other hand, a steel material for automobiles is plastically formed into a desired shape by press forming, and such formed steel parts are assembled into a car body by welding, for example, spot welding. Therefore, a steel material for automobiles is not only required to have press formability, but is also required to be capable of being appropriately welded in various steel sheet assemblies.

However, there is a problem that as the strength of steel materials increases, a phenomenon called delayed fracture is more likely to occur. Delayed fracture refers to a phenomenon in which, when a steel material is subjected to a static load over a period of time, a brittle fracture occurs suddenly without any significant apparent plastic deformation, and herein particularly refers to a hydrogen embrittlement fracture caused by entry of hydrogen into a steel material. Known factors influencing such delayed fracture include the susceptibility of the steel material to hydrogen embrittlement, an applied load (e.g., residual stress), and the amount of hydrogen in the steel material. For example, an increase in the strength of a steel material significantly increases the susceptibility of the steel material to hydrogen embrittlement, which may result in a fracture (cracking) of the steel material.

In particular, a steel material with a tensile strength of 1180 MPa or more has a remarkably increased susceptibility to hydrogen embrittlement (Non-Patent Literature 1). Therefore, a steel material is required to be free from delayed fracture, that is, being excellent in delayed fracture resistance, despite the increase in the strength required for the steel material. In particular, a weld of a steel material has a higher strength than the base steel material, and therefore is more susceptible to hydrogen embrittlement. A demand therefore exists for a method to accurately and quantitatively evaluate delayed fracture properties of a weld of a steel material. It is considered especially important to accurately quantify the effects of the amount of hydrogen in a steel material and an applied load on delayed fracture properties.

In a conventional evaluation method (called gap test method) as disclosed in Non-Patent Literature 2, resistance spot welding test specimens with a gap, having varying nugget diameters, are produced. Hydrogen is introduced into each test specimen by immersing it in hydrochloric acid to evaluate delayed fracture properties. A resistance spot welding test specimen with a gap for use in this test method is produced by placing a spacer steel sheet between two overlapping steel sheets at their both ends to form a gap between the two steel sheets, and subjecting a central gap portion of the overlapping steel sheets to resistance spot welding. A reaction force that recovers the gap acts on a nugget portion of the steel sheets. Therefore, by reducing the nugget diameter, the nugget area that receives the reaction force can be reduced, and the stress (residual stress) applied to the weld can be increased. Therefore, the relationship between the type of a steel sheet and delayed fracture properties can be evaluated by preparing test specimens having varying nugget diameters, introducing hydrogen into the test specimens, and determining the minimum nugget diameter at which delayed fracture (cracking) does not occur in the test specimen.

Non-Patent literature 3 describes a method which involves producing a test specimen by spot-welding high-tensile steel sheets together, introducing diffusible hydrogen into the test specimen, and subjecting the test specimen to a tensile shear test to quantitatively evaluate the amount of diffusible hydrogen, which is a factor influencing delayed fracture properties, and the tensile shear strength.

Citation List

Non-Patent Literature

NPL 1: Shinsaku Matsuyama: "Delayed Fracture", Nikkan Kogyo Shimbun, Tokyo, (1989)
NPL 2: Proceedings of the Japan Welding Society, 37, 3, 125-132 (2019)
NPL 3: Journal of the Japan Institute of Metals and Materials, 85, 2, 75-83 (2021)

Summary of Invention

Technical Problem

The gap test method described in Non-Patent Literature 2 has the following problem. A spot weld of a test specimen is composed of a nugget and a heat-affected zone, which have a microstructure different from that of the base material. A change in the nugget diameter causes a change in the residual stress and microstructure introduced e.g., by a thermal effect during welding. Thus, the gap test method performs an evaluation of delayed fracture properties using test specimens with varying susceptibilities to hydrogen embrittlement and varying applied loads (residual stresses), which are factors influencing delayed fracture. Accordingly, while the gap test method can evaluate, to some extent, the tendency of superiority or inferiority in the delayed fracture properties of a weld among different materials, the influence of the applied load is involved in the evaluation. Thus, the gap test method cannot quantitatively evaluate hydrogen-induced delayed fracture properties of a weld.

The method for evaluating delayed fracture properties described in Non-Patent Literature 3 has the following problem. Test specimens were produced by spot-welding L-shaped steel sheets together, and their tensile shear strengths were evaluated. As a result, in some cases, the tensile shear strength was higher when the amount of hydrogen introduced into a test specimen was large than when the amount of hydrogen introduced into a test specimen was small. Thus, it was found that a tensile shear strength as measured by the method described in Non-Patent Literature 3 sometimes cannot accurately evaluate the delayed fracture properties of a spot weld.

The present invention has been made in view of the above. It is therefore an object of the present invention to provide a method for evaluating delayed fracture properties of a weld of a metal material, which can quantitatively evaluate hydrogen-induced delayed fracture properties of the weld of the metal material.

Solution to Problem

The present inventors, through their intensive studies, found that the above object can be achieved by the following features, leading to completion of the present invention.

[1] A method for evaluating delayed fracture properties of a weld of a metal material, comprising:

a hydrogen introduction step of introducing hydrogen into a test material composed of two or more metal materials having a weld;
a tensile load application step of applying a tensile load to the test material into which hydrogen has been introduced and acquiring a displacement-load curve; and
a first evaluation step of evaluating a crack initiation load based on the displacement-load curve.

[2] The method for evaluating delayed fracture properties of a weld of a metal material according to [1], wherein the weld is a spot weld, and a nugget diameter of the spot weld is $1.7\sqrt{t}$ or more, t being a thickness of the metal material.
[3] The method for evaluating delayed fracture properties of a weld of a metal material according to [1] or [2], wherein a tensile speed in the tensile load application step is $3.0 \times 10^{-4}$ mm/s or less.
[4] The method for evaluating delayed fracture properties of a weld of a metal material according to any one of [1] to [3], further comprising a hydrogen amount measurement step of measuring an amount of hydrogen in the test material into which hydrogen has been introduced in the hydrogen introduction step.
[5] The method for evaluating delayed fracture properties of a weld of a metal material according to [4], further comprising a second evaluation step of evaluating a crack initiation critical load, at which the crack initiation load is constant regardless of the amount of hydrogen in the test material, based on a relationship between the amount of hydrogen in the test material, obtained in the hydrogen amount measurement step, and the crack initiation load obtained in the first evaluation step. Advantageous Effects of Invention

According to the present invention, delayed fracture properties of a weld of a metal material can be evaluated quantitatively with high accuracy.

Brief Description of Drawings

[FIG. 1] FIG. 1 shows (a) a schematic diagram illustrating a tensile test of a test material having an L-joint shape and (b) a diagram showing the results of the tensile test.
[FIG. 2] FIG. 2 is a diagram showing the results of the tensile test of the test material having an L-joint shape, performed after introducing hydrogen into the test material.

[FIG. 3(a)] FIG. 3(a) is a schematic cross-sectional view of a weld.

[FIG. 3(b)] FIG. 3(b) shows diagrams showing the results of a cross-sectional observation of the test material having an L-joint shape, performed after introducing hydrogen into the test material.

[FIG. 4] FIG. 4 is a diagram showing the relationship between the amount of hydrogen at fracture and the crack initiation load in the test material having an L-joint shape.

[FIG. 5] FIG. 5 is a diagram showing the effect of the tensile speed on the crack initiation load of the test material having an L-joint shape.

[FIG. 6] FIG. 6 is a schematic diagram illustrating introduction of hydrogen into the test material having an L-joint shape and a tensile test of the test material. Description of Embodiments

First, the present inventors prepared a test material (L-joint shape) by spot-welding two L-shaped steel sheets together, and performed a tensile shear test on the test material with and without hydrogen being introduced into the test material, and made a detailed comparison and study of the behavior of a displacement-load curve obtained in the tensile shear test.

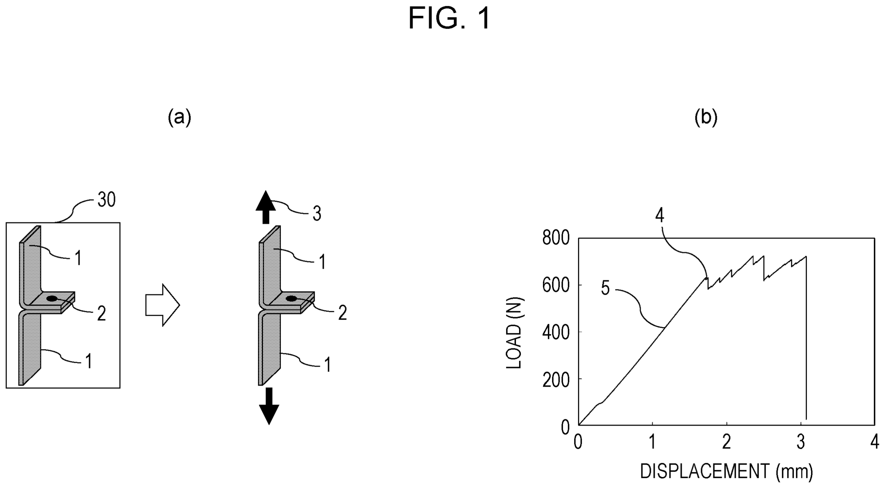

FIG. 1(a) shows a schematic diagram illustrating a tensile test of the test material having an L-joint shape into which no hydrogen has been introduced, and FIG. 1(b) shows a displacement-load curve. The tensile test is performed while applying a load in the tensile direction 3 to the test material 30 having an L-joint shape, composed of two L-shaped steel sheets (metal materials 1) welded together. As can be seen in FIG. 1(b), the load initially increases linearly with increase in the displacement and, at a certain displacement, the load decreases slightly. As will be described later, at displacement 4 where the load decreases slightly, separation (corona bond separation) occurs at a corona bond. The load then increases linearly again with increase in the displacement and, at a certain displacement, the load suddenly drops greatly. This sudden significant drop in the load is believed to be due to the separation at the corona bond (corona bond separation) reaching a nugget and causing a crack in the nugget. Therefore, in the displacement-load curve 5 in the case of not introducing hydrogen into the test material 30, a load value (at which the drop of load occurs) at which the load suddenly drops greatly with increase in the displacement may be used as the fracture strength of the weld in evaluating the properties of the material.

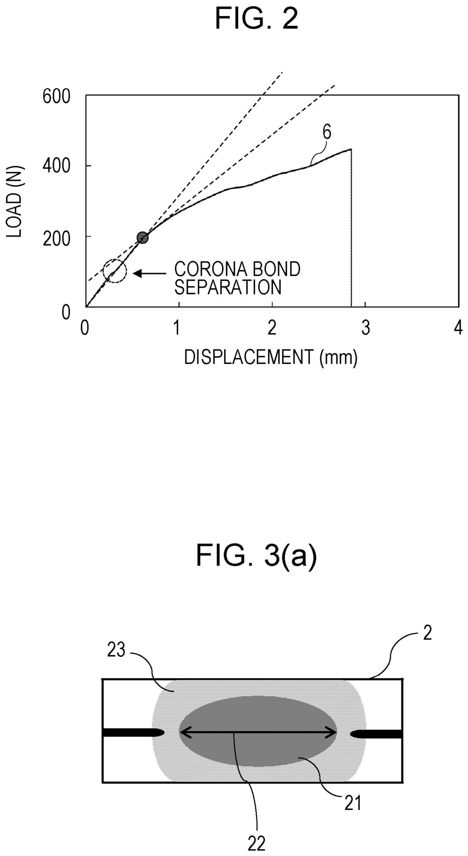

On the other hand, FIG. 2 is a diagram showing the results of the tensile test of the test material 30 having an L-joint shape, performed after introducing hydrogen into the test material 30. As can be seen in FIG. 2, the displacement-load curve 6 in the case of introducing hydrogen into the test material 30 is free of the phenomenon of a sudden significant drop in the load, shown in FIG. 1. Therefore, the present inventors believed that it is not possible to evaluate superiority or inferiority among metal materials using the above-described index, the fracture strength of a weld, and that it is important to perform an evaluation using a new index.

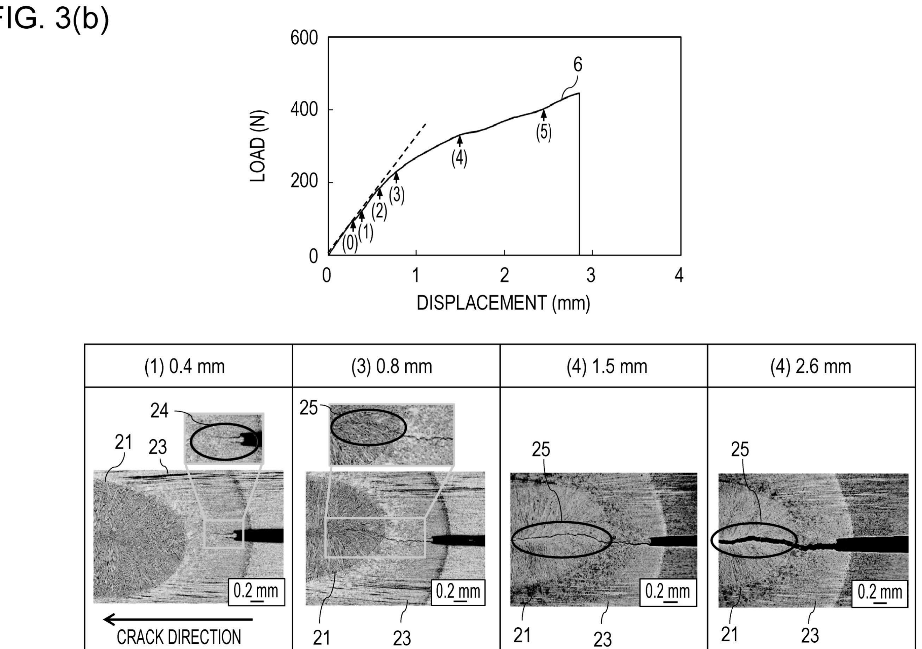

The present inventors interrupted the tensile shear test at various points on the displacement-load curve 6 of FIG. 2, and observed a cross-section of a weld 2 of the test material 30 in detail. FIG. 3(a) shows a schematic cross-sectional view of the weld 2, and FIG. 3(b) shows the results of the cross-sectional observation of the weld 2 at four points (1), (3), (4), and (5) on the displacement-load curve 6. The weld 2 includes a nugget 21, which is a weld metal, and a heat-affected zone 23. The major axis of the nugget 21 is called the nugget diameter 22. FIG. 3(b) (1) shows the results of the cross-sectional observation at a displacement which is slightly larger than a displacement ((0) in FIG. 3(b)) where the load decreased slightly. The results indicate that corona bond separation 24 has occurred in the heat-affected zone 23. From this, it can be estimated that the displacement ((0) in FIG. 3(b)) where the load decreased slightly is the displacement at which the corona bond separation 24 initiates. The displacement ((0) in FIG. 3(b)) where the load decreased slightly is referred to as the displacement at a first gradient change point on the displacement-load curve 6 extending from the origin (displacement 0) to the displacement (fracture displacement) at which fracture occurs. A gradient change point refers to a point where the inclination of the displacement-load changes. FIG. 3(b) (3) shows the results of the cross-sectional observation at a displacement which is slightly larger than a displacement ((2) in FIG. 3(b)) at a gradient change point between the displacement at which the corona bond separation 24 occurs (the displacement at the first gradient change point) and the displacement (fracture displacement) at which fracture occurs in the displacement-load curve 6. As shown in FIG. 3(b) (3), a crack 25 develops within the nugget 21 and, as shown in FIG. 3(b) (4), the crack 25 that has occurred within the nugget 21 further develops with increase in the displacement and, as shown in FIG. 3(b) (5), the crack 25 develops into a crack (fracture) and breakage.

The displacement at the gradient change point between the displacement at which the corona bond separation 24 occurs (the displacement at the first gradient change point) and the displacement (fracture displacement) at which fracture occurs in the displacement-load curve 6 is herein referred to as the displacement at a second gradient change point on the displacement-load curve 6 extending from the origin (displacement 0) to the displacement (fracture displacement) at which fracture occurs. Compared to the inclination of the displacement-load at point (1) where only the corona bond separation 24 has occurred, the inclination of the displacement-load is smaller at point (3) where the crack 25 has occurred in the nugget 21. This is presumably due to a difference in mechanism between them. The present inventors considered that the displacement at the second gradient change point where the inclination of the curve changes is the displacement at which the mechanism changes, that is, the displacement at which the crack 25 begins to occur in the nugget 21. Thus, it is believed, from such behaviors, that in the test specimen into which hydrogen has been introduced, the corona bond separation 24 develops during the period from the displacement at which the corona bond separation 24 occurs (the displacement at the first gradient change point) to the displacement at the second gradient change point, the corona bond separation 24 reaches the nugget 21 at or near the displacement at the second gradient change point, and the crack 25 occurs in the nugget 21 at or near the displacement at the second gradient change point and the crack develops.

Thus, it has been found that it is essential to evaluate the load at the second gradient change point on the displacement-load curve 6, extending from the origin to the fracture displacement, as a fracture strength (also referred to as a crack initiation load) of the weld 2 for hydrogen-induced delayed fracture, and that the use of the crack initiation load makes it possible to quantitatively evaluate hydrogen-induced delayed fracture properties.

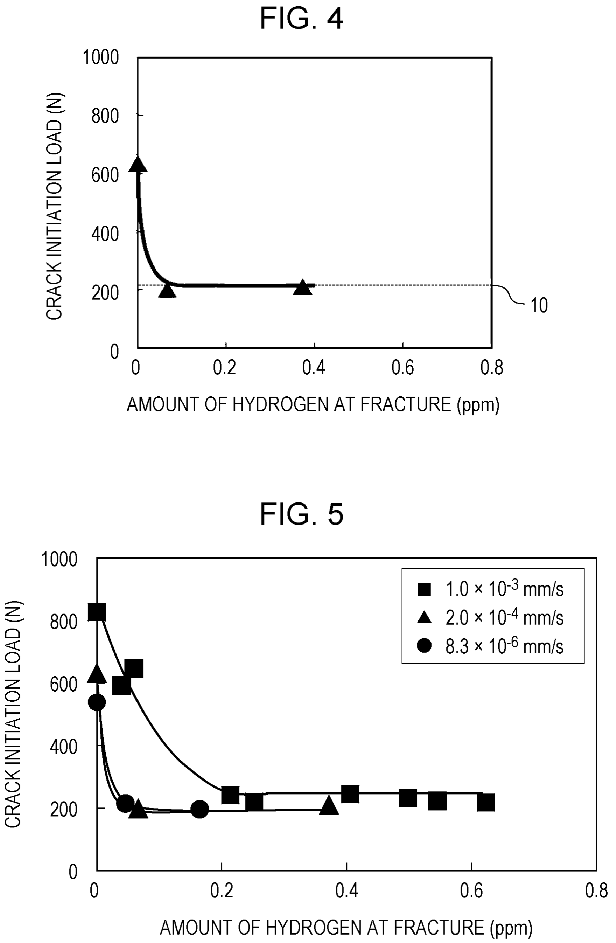

Further, based on the finding that the use of the crack initiation load can quantitatively evaluate the hydrogen-induced delayed fracture properties of the weld 2, the present inventors conducted an experiment, using varying amounts of hydrogen introduced into the test material, to evaluate the relationship between the crack initiation load and the amount of hydrogen at cracking (fracture). The results are shown in FIG. 4. The results in FIG. 4 revealed that the crack initiation load first decreases with increase in the amount of hydrogen introduced into the test specimen, and comes to take a constant value regardless of the amount of hydrogen, and that the constant value varies among steel types. Based on the experimental results, the present inventors considered that by using the constant value (referred to as the crack initiation critical load 10) as an index of the hydrogen-induced delayed fracture properties of the weld 2, it becomes possible to quantitatively determine superiority or inferiority in the hydrogen-induced delayed fracture properties of the weld among types of the metal material 1, in the case of steel material, for example, among steel types.

Embodiments of the method for evaluating delayed fracture properties of a weld of a metal material according to the present invention will now be described. It should be noted that the present invention is not limited to the following embodiments. It should also be noted that components or elements in the following embodiments include those that can be easily replaced by a person skilled in the art, or those that are substantially the same.

A method for evaluating delayed fracture properties of a weld of a metal material according to an embodiment of the present invention comprises: a hydrogen introduction step of introducing hydrogen into a test material 30 composed of two or more metal materials 1 having a weld 2; a tensile load application step of applying a tensile load at a constant speed to the test material into which hydrogen has been introduced; and a first evaluation step of evaluating an crack initiation load based on a displacement-load curve obtained in the tensile load application step.

(Hydrogen Introduction Step)

In the hydrogen introduction step, hydrogen is introduced into a test material composed of two or more metal materials 1 having a weld 2.

Each metal material 1 is not particularly limited as long as it is weldable and exhibits hydrogen-induced delayed fracture properties; however, in the case of a steel material, it is preferably a high-strength steel material, in particular a steel material having a tensile strength of 1180 MPa or more, because as described above, a steel material with a tensile strength of 1180 MPa or more has a remarkably increased susceptibility to hydrogen embrittlement. The tensile strength is more preferably 1320 MPa or more. While the upper limit of the tensile strength is not particularly limited, it is preferred to use a steel material having a tensile strength of 2000 MPa or less. The metal materials 1 may include a coated steel sheet(s) comprising the above high-strength steel material as a base material.

The composition of the steel material is not particularly limited. An exemplary composition comprises, in mass %, C: 0.1 to 0.4%, Si: 0 to 3.0%, Mn: 1 to 10%, P: 0 to 0.05%, S: 0 to 0.005%, with the balance being Fe and incidental impurities. The composition may further contain one or more of Cu, Ti, V, Al, Cr, Ni, etc.

Examples of commercially available steel materials having the above-described tensile strength include JFE-CA1180, JFE-CA1370, JFE-CA1470, JFE-CA1180SF, JFE-CA1180Y1, and JFE-CA1180Y2 (all manufactured by JFE Steel Corporation).

While the thickness of the steel material (base steel sheet) that serves as a substrate in the present invention is not particularly limited, it is preferably about 0.8 to 2.5 mm, more preferably about 1.2 to 2.0 mm.

A welding method for forming the weld 2 is not particularly limited as long as it can weld the metal materials 1. The weld 2 is composed of a weld metal and a heat-affected zone 23. When the welding method is spot welding, the welding conditions are not particularly limited as long as they can form a nugget 21, which is a weld metal, in the metal materials 1. The nugget diameter 22 is preferably $1.7\sqrt{t}$ or more when the thickness of each metal material 1 is t mm. When the nugget diameter 22 is $1.7\sqrt{t}$ or more, the reproducibility of the displacement-load curve 6 in a tensile shear test is high, and the crack initiation load can be evaluated with high accuracy. The nugget diameter 22 is more preferably $2.0\sqrt{t}$ or more, even more preferably $3.0\sqrt{t}$ or more, and most preferably $3.5\sqrt{t}$ or more. On the other hand, if the nugget diameter 22 is too large, the crack initiation load is too high, and it is necessary to increase the load of a tensile tester. This leads to an increase in the size of the equipment and an increase in cost. Therefore, the nugget diameter 22 is preferably $10.0\sqrt{t}$ or less. The nugget diameter 22 is more preferably 7.0√t or less, and even more preferably 5.0√t or less.

When the hardness of the nugget 21 region of the weld 2 is less than 200 HV in terms of Vickers hardness, the possibility of the occurrence of delayed fracture is low; therefore, the hardness of the nugget 21 region of the weld 2 is preferably 200 HV or more in terms of Vickers hardness. The hardness is more preferably 250 HV or more in terms of Vickers hardness. The hardness is even more preferably 300 HV or more in terms of Vickers hardness. The hardness is most preferably 350 HV or more in terms of Vickers hardness. While the upper limit of the hardness of the nugget 21 region of the weld 2 is not particularly limited, the hardness of the nugget 21 region of the weld 2 is preferably 550 HV or less in terms of Vickers hardness because if the region is too hard, initial cracking may occur, making it impossible to evaluate cracking due to delayed fracture. The hardness is more preferably 500 HV or less in terms of Vickers hardness. The hardness can be measured by the method described in the Examples below.

The shape of a test material is not particularly limited. It is possible to use, for example, a test material in which overlapping portions of two rectangular metal materials 1, extending in the same direction, are spot-welded together; a test material (having a cross-joint shape) in which overlapping portions of two rectangular metal materials 1, extending in orthogonal directions, are spot-welded together; a test material (having an L-joint shape) in which, as shown in FIG. 1(a), the short sides of two L-shaped metal materials 1, overlapping each other, are spot-welded together; and a test material (having a U shape) in which the bottoms of two U-shaped metal materials 1, overlapping each other, are spot-welded together.

In the present invention, in the below-described tensile load application step, it is necessary to grip a test specimen with a jig in order to apply a tensile load to the test material. The shape of a grip portion of a test material is not particularly limited as long as the jig can be attached to an area other than a spot weld of the test material, and a tensile load can be applied in a desired direction. For example, the grip portion may be an end portion of a test specimen, or may be formed in the end portion. In particular, in a cross-joint shape, a grip portion may be formed in an end portion using, for example, a bolt. In an L-joint shape, the long side may be used as a grip portion. In a test material having a U shape, the two long sides may be used as a grip portion, or a grip portion may be formed by drilling holes in the two long sides, and passing a pin through the holes.

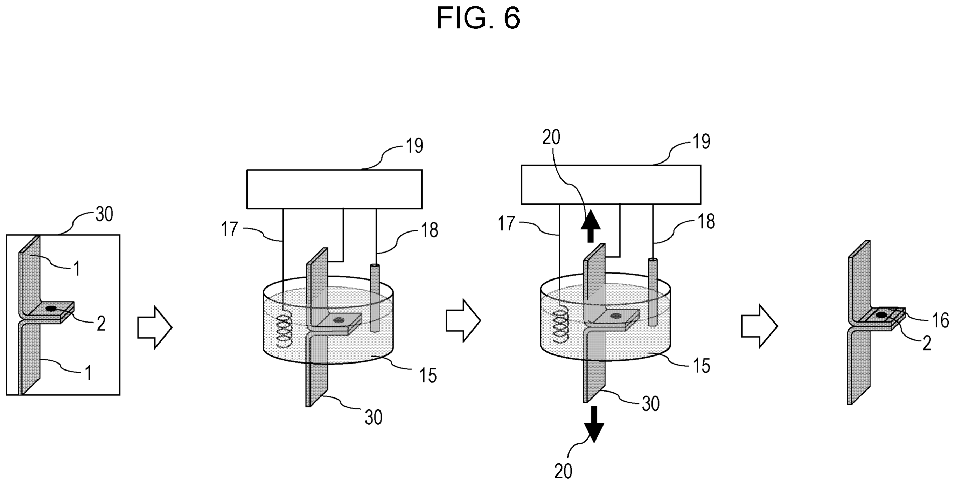

A method for introducing hydrogen into a test material is not particularly limited as long as hydrogen can be introduced into the test material. FIG. 6 is a schematic diagram illustrating introduction of hydrogen into the welded test material 30 having an L-joint shape and a tensile test of the test material 30. Examples of hydrogen introduction methods include a method which involves immersing the test material in an acid, a method which involves cathodic hydrogen charging in an electrolyte solution, a method which involves exposing the test material to high-pressure hydrogen gas, and a method which involves exposing the test material to a corrosive environment. To evaluate the delayed fracture properties of a steel material, it is preferred to use a method which can sufficiently introduce hydrogen into various hydrogen trapping sites in the steel material and, from the viewpoint of controlling the amount of hydrogen to be introduced, it is preferred to use a method involving cathodic hydrogen charging in an electrolyte solution 15. In particular, using the test material 30 as a cathode and a Pt (platinum) electrode 17 or the like as an anode in the electrolyte solution 15, an electric current is passed between the cathode and the anode to electrolyze the solution, and hydrogen generated is introduced into the test material. The cathodic hydrogen charging method can control the amount of hydrogen to be introduced into the test material 30 by, for example, changing the electric current or the potential. The hydrogen introduction step may be performed before the next tensile load application step, or may be continued during the tensile load application step. Reference sign 20 denotes the direction in which a tensile load is applied. Examples of the electrolyte solution 15 include 0.1N NaOH, 1N NaOH + 0.3 g/L $NH_4SCN$, and 3 wt% NaCl + 3 g/L $NH_4SCN$.

(Tensile Load Application Step)

Next, a tensile load is applied to the test specimen into which hydrogen has been introduced.

A common tensile tester may be used to apply a tensile load. FIG. 5 shows the relationship between the amount of hydrogen and the crack initiation load, obtained in an evaluation experiment in which a tensile load was applied to an example spot-welded steel material having an L-joint shape at varying tensile speeds. In the experiment, a cold-rolled steel sheet having a tensile strength of 1470 MPa grade was used, and welding was performed under conditions where the set value of the nugget diameter was 4√t (t is the thickness of the steel sheet), and the measured value was 4.73 mm. The hardness of the nugget 21 region of the resulting weld was 488 HV. The curve showing the relationship between the amount of hydrogen and the crack initiation load, obtained when the tensile speed was $2.0 \times 10^{-4}$ mm/s, is similar to the curve obtained when the tensile speed was $8.3 \times 10^{-6}$ mm/s. On the other hand, the crack initiation load is higher in the curve obtained when the tensile speed was $1.0 \times 10^{-3}$ mm/s. This is presumably because delayed fracture of a steel material is caused by hydrogen penetrating and diffusing into the steel material, and accumulating at defects and stress concentration sources. It is believed that hydrogen often diffuses late into a steel material, and this tendency is more prominent in a high-strength steel material for which delayed fracture is more problematic. Therefore, in order to appropriately evaluate the hydrogen-induced delayed fracture properties of a weld of a steel material, it is preferred to use a slow tensile speed and ensure the time required for hydrogen to penetrate and diffuse into the steel material and accumulate at defects and stress concentration sources. Thus, the tensile speed is preferably $3.0 \times 10^{-4}$ mm/s or less. The tensile speed is more preferably $2.0 \times 10^{-4}$ mm/s or less, even more preferably $1.0 \times 10^{-4}$ mm/s or less, and most preferably $1.0 \times 10^{-5}$ mm/s or less. On the other hand, the lower limit of the tensile speed is not particularly limited; however, in order to avoid an excessively long test time, the tensile speed is preferably $6.0 \times 10^{-7}$ mm/s or more. The tensile speed is even more preferably $1.0 \times 10^{-6}$ mm/s or more, and even more preferably $3.0 \times 10^{-6}$ mm/s or more. The present step last acquires a displacement-load curve.

(First Evaluation Step)

Next, a crack initiation load is evaluated from the displacement-load curve 6 obtained in the tensile load application step. The crack initiation load is a load at which a crack begins to occur in a weld metal, and in the case of spot welding, is a load at which a crack begins to occur in a nugget which is a weld metal. A first gradient change point is herein defined as a point of a first change in the gradient of the displacement-load curve 6 shown in FIG. 3(b), extending from the origin (displacement 0) to the displacement (fracture displacement) at which fracture occurs. The displacement at the first gradient change point is a displacement where the load is slightly lower, and is a displacement at which corona bond separation occurs.

A second gradient change point ((2) in FIG. 3(b)) is herein defined as a point of a change in the gradient of the displacement-load curve 6 in its portion ranging from the displacement at which corona bond separation occurs to the fracture displacement.

The crack initiation load in the present invention can be evaluated using the second gradient change point on the displacement-load curve 6, in particular a load within $\pm 1\%$ of the load at the second gradient change point. The load at which a crack begins to occur in a weld metal (nugget in the case of spot welding) is identical to the load within $\pm 1\%$ of the load at the second gradient change point which is a gradient change point on the displacement-load curve 6 in its portion ranging from the displacement at which corona bond separation occurs to the fracture displacement, shown in FIG. 3(b).

In another embodiment of the present invention, a hydrogen amount measurement step for measuring the amount of hydrogen may be provided in order to evaluate the relationship between the amount of hydrogen in the test specimen 30 and the crack initiation load.

(Hydrogen Amount Measurement Step)

In the hydrogen amount measurement step, the amount of hydrogen in the test material 30, into which hydrogen has been introduced in the hydrogen introduction step, is measured. To perform the measurement in this step, a test specimen 16 for hydrogen amount measurement is prepared. The test specimen 16 may be cut from the test material 30, which has fractured in the tensile load application step, such that it includes a weld 2. Alternatively, the test specimen 16 for hydrogen amount measurement may be prepared in the following manner: A test material 30 is produced separately (under the same conditions as those for a test material for tensile test), and hydrogen is introduced into the test material 30 in the hydrogen introduction step under the same conditions as those for a test material for tensile test. The test specimen 16 is cut from the test material 30 (the test material before the tensile load application step) such that it includes a weld 2. The former method for preparing the test specimen 16 for hydrogen amount measurement is preferred. The test specimen 16 for hydrogen amount measurement is immersed and stored in liquid nitrogen so that hydrogen will not be desorbed. When measuring the amount of hydrogen, the test specimen 16 for hydrogen amount measurement is taken from the liquid nitrogen, and measurement of the amount of hydrogen is preferably performed quickly. Thermal desorption analysis, for example, can be used as a method for measuring the amount of hydrogen. The hydrogen amount measurement step may be performed at any time after the hydrogen introduction step of introducing hydrogen into the test material.

In another embodiment of the present invention, a second evaluation step for evaluating the crack initiation critical load 10 from the relationship between the amount of hydrogen, obtained in the hydrogen amount measurement step, and the crack initiation load, obtained in the first evaluation step, may be provided so that superiority or inferiority in hydrogen-induced delayed fracture properties among metal materials can be quantitatively determined.

(Second Evaluation Step)

The second evaluation step evaluates the crack initiation critical load 10, at which the crack initiation load is constant regardless of the amount of hydrogen, from the relationship between the amount of hydrogen, obtained in the hydrogen amount measurement step, and the crack initiation load obtained in the first evaluation step. The evaluation is preferably performed at a constant nugget diameter 22; the nugget diameter 22 preferably has a variation (mm) within the range of $\pm 0.3\sqrt{t}$, t being the thickness of the metal material. This is because a change in the nugget diameter 22 causes a change in the residual stress and microstructure introduced, for example, by a thermal effect. It is therefore possible that factors other than hydrogen may affect the delayed fracture properties. The variation of the nugget diameter 22 is more preferably within the range of $\pm0.2\sqrt{t}$. While the lower limit of the variation range (mm) of the nugget diameter 22 is not particularly limited, the variation range is preferably not less than $0.05\sqrt{t}$. As described above, the present inventors have found that when the amount of hydrogen and the crack initiation load are plotted, the crack initiation load takes an approximately constant value in a hydrogen amount range from a certain value. In the present invention, the crack initiation critical load (reference sign 10 in FIG. 4) is defined as the constant value at which the crack initiation load is constant regardless of the amount of hydrogen. The present inventors have also found that the crack initiation critical load 10 takes different values among different types of metal materials 1, for example, in the case of the steel material, different types of steels, which makes it possible to quantitatively determine superiority or inferiority in the hydrogen-induced delayed fracture properties of a weld 2 of a metal material 1. Thus, the use of the crack initiation critical load 10 can quantitatively determine superiority or inferiority in the hydrogen-induced delayed fracture properties of a weld 2 of a metal material 1.

EXAMPLES

The following examples illustrate the present invention specifically. It should be noted that the present invention is not limited to the examples.

(Hydrogen Introduction Step)

First, a steel sheet, having the chemical composition of main components, the thickness and the tensile strength shown in Table 1, was cut into a rectangular piece of 150 mmC $\times$ 30 mmL (C represents the width direction of the steel sheet, and L represents the longitudinal direction of the steel sheet), and the long sides of the rectangular piece were bent at right angle to produce an L-shaped steel sheet as shown in FIG. 6.
Thereafter, the short sides of two such L-shaped steel sheets were placed back-to-back, and spot-welded under the below-described conditions to produce a test material 30 (having an L-joint shape, see FIG. 6). The test material 30 was produced for each of steel sheets A to C. The spot welding was performed under the following conditions. The spot welding was performed at room temperature while keeping electrodes always water-cooled. DR-type electrodes made of chromium copper, having a tip diameter of 6 mm and a curvature radius of 40 mm, were used as lower and upper welding electrodes. The spot welding was performed under conditions where the welding current was in the range of 3.0 to 4.5 kA and the nugget diameter was set to a value shown in Table 2 or 3. The Tables also show nugget diameters that were actually measured.
Subsequently, the test material 30 as a cathode, a Pt electrode 17 as an anode, and a silver-silver chloride (Ag/AgCl) electrode 18 as a reference electrode were connected to a potentiostat 19, and hydrogen was introduced into the test material 30 by potentiostatic control of the test material 30. The amount of hydrogen was varied by controlling the type and potential of an electrolyte solution 15. 1N NaOH + 0.3 g/L $NH_4CN$ or 0.1N NaOH was used as the electrolyte solution 15, and the potential was set in the range of -1000 to -1500 mV vs. SHE. The next tensile load application step was performed while continuing the cathodic hydrogen charging process for introducing hydrogen.

(Tensile Load Application Step)

A common tensile tester was used to apply a tensile load to the test material 30. The tensile speed during the application of the tensile load is described in Table 2.

(First Evaluation Step)

A crack initiation load was evaluated in the displacement-load curve obtained in the tensile load application step. The load corresponding to the second gradient change point on the displacement-load curve, extending from the origin (displacement 0) to the fracture displacement, was taken as a crack initiation load. An example of the results obtained is shown in Table 2.
As can be seen in Table 2, the use of the index, the crack initiation load, can quantitatively evaluate hydrogen-induced delayed fracture properties under certain conditions in the hydrogen introduction step.

(Hydrogen Amount Measurement Step)

A test specimen 16 for hydrogen amount measurement, having a size of 10 $\times$ 30 mm and including a weld 2, was cut out from the test specimen 30 that had fractured in the tensile load application step. In order to prevent desorption of hydrogen that had been introduced into the test specimen 30, the test specimen 16 for hydrogen amount measurement was introduced into liquid nitrogen and quenched. Thereafter, the quenched test specimen 16 for hydrogen amount measurement was promptly subjected to thermal desorption analysis to measure the amount of hydrogen. A low-temperature heating-type hydrogen analyzer was used for the thermal desorption analysis. The thermal desorption analysis was performed at a heating rate of 200°C/h in the temperature range from -50°C to 800°C. An integrated value of the amount of hydrogen as measured in the temperature range from -50 to 200°C was used as the amount of diffusible hydrogen. The hydrogen amount measurement step may be performed at any time after the hydrogen introduction step of introducing hydrogen into the test material.

(Second Evaluation Step)

In order to quantitatively evaluate superiority or inferiority among metal materials, a second evaluation step was performed. First, for each of test materials 30 having the same nugget diameter, the amount of hydrogen introduced was varied, the crack initiation load and the amount of hydrogen were determined by the above procedure, and the relationship between them was plotted. An example is shown in FIG. 4. As shown in FIG. 4, the load at which the crack initiation load is constant with respect to the amount of hydrogen was taken as a crack initiation critical load. The steel sheet, nugget diameter, and Vickers hardness of FIG. 4 are the same as those shown in FIG. 5, and the tensile speed is $2.0 \times 10^{-4}$ mm/s.

Subsequently, the amount of the crack initiation critical load was evaluated for the steel sheets A, B, and C. The results are shown in Table 3. If the order of superiority in hydrogen-induced delayed fracture properties among metal materials, obtained by the method for evaluating delayed fracture properties of a weld of a metal material according to the present invention, matches the order of superiority in the delayed fracture properties of a weld among metal materials, obtained by the conventional gap test method, then the evaluation method of the present invention was judged to have been able to appropriately evaluate the delayed fracture properties of a weld.

The order of superiority in the delayed fracture resistance among the steel sheets A, B, and C, obtained by the conventional gap test method, was as follows: (superior) steel sheet A > steel sheet B > steel sheet C (inferior).

By using the method for evaluating delayed fracture properties of a weld of a metal material according to the present invention, the delayed fracture properties of a weld can be evaluated using a test material having the same nugget diameter based on Table 3. This makes it possible to eliminate load stress as much as possible, and to quantitatively evaluate the hydrogen-induced delayed fracture properties of a weld. Thus, superiority or inferiority in the delayed fracture properties among materials can be appropriately evaluated quantitatively.

(Evaluation of Vickers Hardness)

The Vickers hardness of a weld 2 was measured by pressing a square pyramidal diamond indenter into the surface of the weld at a load of 300 g to form an indentation, and calculating the Vickers hardness by the following formula using the load and the average length of the diagonal of the indentation. 20 points were measured at 0.2-mm intervals in an area from one end of a nugget to the center of the weld using the above method, and the average value was used as the Vickers hardness. The measured values are shown in Tables 2 and 3.

Vickers hardness = constant $\times$ (test force/surface area of indentation)

[Table 1]

| Symbol | Steel type | Thickness mm | Tensile strength MPa | Chemical composition / mass % | | | | |
|---|---|---|---|---|---|---|---|---|
| | | | | C | Si | Mn | S | P |
| 1 | Steel sheet A | 1.4 | 1490 | 0.20 | 0.24 | 1.23 | 0.0005 | 0.0082 |
| 2 | Steel sheet B | 1.4 | 1211 | 0.11 | 0.52 | 2.50 | 0.0006 | 0.0090 |
| 3 | Steel sheet C | 1.4 | 980 | 0.15 | 0.20 | 4.98 | 0.0008 | 0.0034 |

[Table 2]

| Symbol | Steel type | Nugget diameter | | Tensile speed (mm/s) | Average Vickers hardness of weld (HV) | Conditions of cathodic hydrogen charging method | | Crack initiation load (N) |
|---|---|---|---|---|---|---|---|---|
| | | Set value | Measured value (mm) | | | Electrolyte solution | Potential (mV vs. SSE) | |
| 1 | Steel sheet A | $2.0\sqrt{t}$ | 2.39 | $1.0 \times 10^{-6}$ | 451 | 1N NaOH + 0.3 g/L $NH_4SCN$ | -1500 | 350 |
| 2 | Steel sheet A | $2.0\sqrt{t}$ | 2.29 | $1.0 \times 10^{-6}$ | 432 | 1N NaOH + 0.3 g/L NH4SCN | -1200 | 365 |
| 3 | Steel sheet A | $2.0\sqrt{t}$ | 2.37 | $1.0 \times 10^{-6}$ | 460 | 0.1N NaOH | -1100 | 600 |

[Table 3]

| Symbol | Steel type | Nugget diameter | | Tensile speed (mm/s) | Average Vickers hardness of weld (HV) | Crack initiation critical load (N) | Order of superiority in delayed fracture resistance |
|---|---|---|---|---|---|---|---|
| | | Set value | Measured value (mm) | | | | |
| 1 | Steel sheet A | $2.0\sqrt{t}$ | 2.35 | $1.0\times10^{-6}$ | 417 | 350 | Steel sheet A>Steel sheet B>Steel sheet C |
| 2 | Steel sheet B | $2.0\sqrt{t}$ | 2.34 | $1.0\times10^{-6}$ | 338 | 300 | |
| 3 | Steel sheet C | $2.0\sqrt{t}$ | 2.36 | $1.0\times10^{-6}$ | 276 | 200 | |
| 4 | Steel sheet A | $3.0\sqrt{t}$ | 3.53 | $1.0\times10^{-4}$ | 447 | 348 | Steel sheet A>Steel sheet B>Steel sheet C |
| 5 | Steel sheet B | $3.0\sqrt{t}$ | 3.54 | $1.0\times10^{-4}$ | 417 | 312 | |
| 6 | Steel sheet C | $3.0\sqrt{t}$ | 3.50 | $1.0\times10^{-4}$ | 369 | 210 | |
| 7 | Steel sheet A | $3.8\sqrt{t}$ | 4.99 | $1.5\times10^{-4}$ | 448 | 358 | Steel sheet A>Steel sheet B>Steel sheet C |
| 8 | Steel sheet B | $3.8\sqrt{t}$ | 4.94 | $1.5\times10^{-4}$ | 414 | 305 | |
| 9 | Steel sheet C | $3.8\sqrt{t}$ | 4.64 | $1.5\times10^{-4}$ | 378 | 210 | |
| 10 | Steel sheet A | $5.3\sqrt{t}$ | 6.33 | $7.0\times10^{-3}$ | 444 | 327 | Steel sheet A>Steel sheet B>Steel sheet C |
| 11 | Steel sheet B | $5.3\sqrt{t}$ | 6.37 | $7.0\times10^{-3}$ | 417 | 312 | |
| 12 | Steel sheet C | $5.3\sqrt{t}$ | 6.28 | $7.0\times10^{-3}$ | 367 | 250 | |

Reference Signs List

1 metal material
2 weld
3 tensile direction
4 displacement
5 displacement-load curve in the case of not introducing hydrogen into a test material
6 displacement-load curve in the case of introducing hydrogen into a test material
10 crack initiation critical load
15 electrolyte solution
16 test specimen for hydrogen amount measurement
17 Pt (platinum) electrode

18 silver-silver chloride (Ag/AgCl) electrode
19 potentiostat
20 direction in which a tensile load is applied
21 nugget
22 nugget diameter
23 heat-affected zone
24 corona bond separation
25 crack
30 test material, test specimen

## Claims

1. A method for evaluating delayed fracture properties of a weld of a metal material, comprising:

   a hydrogen introduction step of introducing hydrogen into a test material composed of two or more metal materials having a weld;
   a tensile load application step of applying a tensile load to the test material into which hydrogen has been introduced and acquiring a displacement-load curve; and
   a first evaluation step of evaluating a crack initiation load based on the displacement-load curve.

2. The method for evaluating delayed fracture properties of a weld of a metal material according to claim 1, wherein the weld is a spot weld, and a nugget diameter of the spot weld is $1.7\sqrt{t}$ or more, t being a thickness of the metal material.

3. The method for evaluating delayed fracture properties of a weld of a metal material according to claim 1 or 2, wherein a tensile speed in the tensile load application step is $3.0 \times 10^{-4}$ mm/s or less.

4. The method for evaluating delayed fracture properties of a weld of a metal material according to any one of claims 1 to 3, further comprising a hydrogen amount measurement step of measuring an amount of hydrogen in the test material into which hydrogen has been introduced in the hydrogen introduction step.

5. The method for evaluating delayed fracture properties of a weld of a metal material according to claim 4, further comprising a second evaluation step of evaluating a crack initiation critical load, at which the crack initiation load is constant regardless of the amount of hydrogen in the test material, based on a relationship between the amount of hydrogen in the test material, obtained in the hydrogen amount measurement step, and the crack initiation load obtained in the first evaluation step.

# FIG. 1

(a)

(b)

# FIG. 2

# FIG. 3(a)

FIG. 3(b)

LOAD (N)

600

400

200

0

0    1    2    3    4

DISPLACEMENT (mm)

6

(0)(1)(2)(3)(4)(5)

| (1) 0.4 mm | (3) 0.8 mm | (4) 1.5 mm | (4) 2.6 mm |

(1) 0.4 mm — 24, 23, 21, 0.2 mm, CRACK DIRECTION

(3) 0.8 mm — 25, 23, 21, 0.2 mm

(4) 1.5 mm — 25, 23, 21, 0.2 mm

(4) 2.6 mm — 25, 23, 21, 0.2 mm

## FIG. 4

## FIG. 5

# FIG. 6

EP 4 700 360 A1

## INTERNATIONAL SEARCH REPORT

| International application No. |
|---|
| **PCT/JP2024/016287** |

**A. CLASSIFICATION OF SUBJECT MATTER**

*G01N 3/00*(2006.01)i; *G01N 17/00*(2006.01)i
FI:   G01N3/00 Q; G01N17/00

According to International Patent Classification (IPC) or to both national classification and IPC

**B. FIELDS SEARCHED**

Minimum documentation searched (classification system followed by classification symbols)

G01N3/00; G01N17/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | 古迫　誠司, 薄鋼板溶接継手の強度信頼性向上に関する研究, 横浜国立大学　博士論文. pp. 41-59, (FURUSAKO, Seiji. Study on strength reliability improvement in welded joints of thin steel sheet.), non-official translation (Yokohama National University, Doctoral Thesis) pp. 41-59 | 1-5 |
| Y | JP 2006-29977 A (JFE STEEL CORPORATION) 02 February 2006 (2006-02-02) paragraphs [0031]-[0035] | 1-5 |
| A | JP 2009-69004 A (NIPPON STEEL CORPORATION) 02 April 2009 (2009-04-02) entire text, all drawings | 1-5 |
| A | WO 2022/249651 A1 (JFE STEEL CORPORATION) 01 December 2022 (2022-12-01) entire text, all drawings | 1-5 |
| A | WO 2015/080261 A1 (MITSUBISHI HEAVY INDUSTRIES, LTD.) 04 June 2015 (2015-06-04) entire text, all drawings | 1-5 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **13 June 2024** | **09 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| | International application No. |
|---|---|
| | **PCT/JP2024/016287** |

### C. DOCUMENTS CONSIDERED TO BE RELEVANT

| Category* | Citation of document, with indication, where appropriate, of the relevant passages | Relevant to claim No. |
|---|---|---|
| A | JP 2022-124790 A (NIPPON STEEL CORPORATION) 26 August 2022 (2022-08-26)<br>entire text, all drawings | 1-5 |

Form PCT/ISA/210 (second sheet) (July 2022)

## INTERNATIONAL SEARCH REPORT
### Information on patent family members

International application No.

**PCT/JP2024/016287**

| Patent document cited in search report | | | Publication date (day/month/year) | Patent family member(s) | | | Publication date (day/month/year) |
|---|---|---|---|---|---|---|---|
| JP | 2006-29977 | A | 02 February 2006 | (Family: none) | | | |
| JP | 2009-69004 | A | 02 April 2009 | (Family: none) | | | |
| WO | 2022/249651 | A1 | 01 December 2022 | EP<br>entire text, all drawings<br>KR 10-2023-0169332<br>CN | 4316719<br><br><br>117320833 | A1<br><br>A<br>A | |
| WO | 2015/080261 | A1 | 04 June 2015 | US<br>entire text, all drawings<br>EP | 2016/0279739<br><br>3075486 | A1<br><br>A1 | |
| JP | 2022-124790 | A | 26 August 2022 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHINSAKU MATSUYAMA**. Delayed Fracture. Nikkan Kogyo Shimbun, 1989
- *Proceedings of the Japan Welding Society*, 2019, vol. 37 (3), 125-132
- *Journal of the Japan Institute of Metals and Materials*, 2021, vol. 85 (2), 75-83